**Europäisches Patentamt**

**European Patent Office**

**Office européen des brevets**

(19)

(11) Publication number : **0 422 072 B1**

# EUROPEAN PATENT SPECIFICATION

(12)

(45) Date of publication of patent specification :
19.05.93 Bulletin 93/20

(51) Int. Cl.⁵ : **C07K 15/00, C12N 15/86**

(21) Application number : **89907535.2**

(22) Date of filing : **07.06.89**

(86) International application number :
**PCT/US89/02486**

(87) International publication number :
**WO 89/12103 14.12.89 Gazette 89/29**

(54) METHOD OF SELECTING FOR RECOMBINANT POX VIRUSES.

(30) Priority : **10.06.88 US 205189**

(43) Date of publication of application :
**17.04.91 Bulletin 91/16**

(45) Publication of the grant of the patent :
**19.05.93 Bulletin 93/20**

(84) Designated Contracting States :
**AT BE CH DE FR GB IT LI**

(56) References cited :
**EP-A- 0 262 043
Proceedings of the National Academy of Sciences of the USA, vol. 82, no. 5, March 1985 (Washington, US), S.L. Mansour et al.: "An adenovirus vector system used to express polyoma virus tumor antigens", pp. 1359-1363**

(73) Proprietor : **APPLIED BIOTECHNOLOGY, INC.
80 Rogers Street
Cambridge Massachusetts 02142 (US)**

(72) Inventor : **SMITH, Kenneth
10 Third Street
Natick, MA 01760 (US)**

(74) Representative : **Holdcroft, James Gerald, Dr. et al
Graham Watt & Co., Riverhead
Sevenoaks, Kent TN13 2BN (GB)**

EP 0 422 072 B1

Jouve, 18, rue Saint-Denis, 75001 PARIS

## Description

### Background of the Invention

Vaccinia virus is the most well-studied type of orthopox virus. Dales and Pogo, (1981), Biology of Pox Viruses, W. Kingsbury and H. Zur Hausen (eds.), In Virology Monographs, Vol. 18, Springer-verlag, New York, NY; and Moss, B. (1985) In: Virology, B.N. Fields (ed), p. 685-704, Raven Press, NY. It contains a genome of about 185 kilobase pairs (kb) which encode most of the necessary proteins for its cytoplasmic replication. Vaccinia virus has become a useful eukaryotic expression vector Panicali, D. and Paoletti, E. (1982), Proc. Natl. Acad. Sci. USA, 79:4927-4931; and M. Mackett et al., (1982), Proc. Natl. Acad. Sci. USA, 79:7415-7419 and has been demonstrated to be an effective live virus vaccine vector for foreign antigens inserted into its genome. G.L. Smith et al., Nature (London), 302:490-495 (1983); and D. Panicali et al., Proc. Natl. Acad. Sci. USA, 80:5364-5368 (1983).

Because of the large size of the vaccinia virus genome and the fact that its DNA is non-infectious, it is impossible to directly clone foreign DNA into vaccinia virus. Rather, in vivo homologous recombination is used to insert foreign DNA into the vaccinia virus genome. Typical in vivo homologous recombination experiments yield 0.1% - 1.0% recombinants. D.D. Spyropoulos et al., J. Virol., 62:1046-1054 (1988). Commonly used recombinant identification and selection systems include DNA replica filter hybridization (E. Nakano et al., Proc. Natl. Acad. Sci, 79:1593-1596 (1982), insertion or deletion of the beta-galactosidase gene along with the visual identification of recombinant plaques using the chromogenic substrate X-gal (10) and thymidine kinase (TK) selection. M. Mackett et al., Proc. Natl. Acad. Sci. USA, 79:7415-7419 (1982); J. Campione-Piccardo et al., J. Virol., 31 281-287 (1979); and D.B. Davis et al., J. Virol., 13:140-145 (1974).

Although plaque hybridization and LacZ expression assays are often used for the construction of recombinants they are not true selections. S. Chakrabarti et al., Mol. Cell Biol., 5:3403-3409 (1985); and D. Panicali et al., Gene, 47:193-199 (1986). Rather, identification of the inserted gene is accomplished using a radioactive nucleir acid probe specific for recombinant sequences or by the use of a chromogenic indicator to visualize recombinants expressing beta-galactosidase. These methods only identify and do not select for recombinants. Further, recombinant plaque purifications using these indicators are more labor intensive than a dominant selectable function like TK.

In order to utilize the TK gene as a positive selection system, a plasmid containing the TK gene, the foreign gene and vaccinia virus flanking sequences is recombined with a TK virus. The resulting TK$^+$ recombinants are selected for using methotrexate selection on Hu143TK$^-$ cells. J. Campione-Piccardo et al., J. Virol., 31:281-287 (1979); and D.B. Davis et al., J. Virol., 13:140-145 (1974). Both the positive and negative TK finase selection systems efficiently select for recombinants. The methotrexate selection is extremely high on the host cells and limits the yield of virus from a plaque upon prolonged exposure to the drug. In addition, 5-bromodeoxy uridine is a potent mutagen and may induce undesirable mutations in the recombinants.

Therefore, a selection system which selects for recombinants without the use of drugs, mutagens, foreign genes or chromogenic indicators would be desirable.

### Summary of the Invention

This invention pertains to a method of selecting for viral recombinants without the use of drugs, mutagens or chromogenic indicators. The method entails the use of a virus which is incapable of replicating in particular "nonpermissive" selective host cells because it lacks the function of a gene necessary for replication in the selective host cell. The virus is allowed to recombine with a donor DNA vector which contains the viral gene necessary for replication in the selective host cell along with a foreign gene to be inserted into the virus, one or more viral promoters controlling expression of the genes and viral flanking sequences which are substantially homologous to a region of the virus genome. The resulting viral recombinants contain the foreign gene and the viral gene required for replication in the selective host cells. This allows the recombinant virus to be selected on the basis of its ability to grow on the selective host cells.

The method of this invention can be used to efficiently select for virus recombinants, particularly pox viruses. This selection system is at least as efficient as previously reported selection systems yet does not require the use of drugs, mutagens, chromogenic indicators or foreign genes.

The invention also pertains to mutant viruses which are incapable of replicating on a selective host cell because they lack the function of gene necessary for replication on the cells. The invention further pertains to donor DNA vectors, into which the gene or genes necessary for replication on the selective host cell have been inserted, for recombination with the mutant virus to produce recombinant virus which are capable of replicating on the host cell. The donor vector also contains a foreign gene under the direction of a pox viral promoter for insertion into the recombinant virus. These vectors can be used to create recombinant viruses which express the foreign gene and which can be selected for by growing on the selective host cell.

Brief Description of the Figures

Figure 1 depicts the restriction maps of the portion of (a) the genome of NYCBH vaccinia virus showing the location of the 29K gene; and (b) the genome of vABT33 vaccinia virus which lacks a portion of the 29K gene and, (c) the genome of vAbT71.

Figure 2 is a schematic representation of the plasmid pAG10 SAG and the portion of the vABT33 viral genome in which it is incorporated during transfection.

Figure 3 depicts the restriction map showing the sites of restriction and construction of the pTK7.5Kgp5029K plasmid.

Figure 4 is a schematic representation of the predicted genomic structures of the vaccinia viruses NYCBH, vABT33 and v30KSAG.

Figure 5 shows the strategy used to construct plasmids pAbT1201 and pAbT1204 which were used to construct vAbT33 and vAbT71 respectively.

Figure 6 shows the construction of plasmid pAbT4572, a plasmid vector for the insertion of the SIV-$_{MAC-251}$ env gene into vaccinia with the 29K gene, with the env gene under the control of the vaccinia 40k promoter.

Figure 7 shows the construction of plasmids pAbT4575 and pAbT4577. PaBt4575 is an in vivo recombinant vector for the insertion of the SIV$_{MAC-251}$ gag-prot gene under the control of the rok promoter. pAbT4577 is an IVR vector for the insertion of the SIV$_{MAC-251}$ env and gag-prot genes into vaccinia with the 29K gene, with the env gene under the control of the 30k promoter and the gag-prot gene under the control of the 40k promoter.

Figure 8 shows the construction of plasmid pAbT4585, an IVR vector for the insertion of the SIV$_{MAC-251}$ env and gag-prot genes into vaccinia, with the env gene under the control of the 40k promoter and the gag-prot gene under the control of the 7.5k promoter.

Detailed Description of the Invention

This invention pertains to a method of selecting for recombinant viruses, particularly pox viruses. According to the method, a pox virus which is incapable of replicating on certain host cells because it lacks a gene necessary for replication on these cells is recombined with a vector containing the gene function necessary for replication. The recombinants are selected by plating the virus on a host cell which is selective for the recombinant virus. Recombinant virus capable of expressing foreign mycobacterial, bacterial, viral parasite antigens can be produced by integrating into the virus genome a gene or genes encoding the antigens of interest, and selecting for the recombinant virus by plaquing the cotransfected virus on cells selective for the recombinants. In a preferred embodiment of the invention, the virus is a pox virus, preferably a mutant vaccinia virus, which lacks the function of the 29K gene. The 29K gene permits virus replication on RK13 (rabbit kidney) cells, as described by Gillard et al. in Proc. Natl. Acad. Sci. USA 83:5573 (1986). This vaccinia mutant is allowed to recombine with a donor DNA vector which contains the 29K gene function and a foreign gene under the direction of a vaccinia promoter sequence, and vaccinia flanking sequences which are not essential for replication. The resulting recombinant virus is selected by plaque assay on selective host cells, such as RK13 cells which are selective for virus having the 29K gene.

1. Pox Viruses

Any member of the pox family which is a mutant virus incapable of replicating on a selective host cell can be used for the generation of the recombinant viruses described herein. Other viruses besides pox viruses can also be used, as long as the virus is incapable of replicating on a selective host cell, and it can be transfected with the gene enabling if to replicate on the selective host cells. For many purposes, including vaccine development, the preferred pox virus is a mutant virus which is non-virulent in normal humans and animals. For example, for humans and other mammals, the preferred pox virus is vaccinia virus, a relatively benign virus which has been used for years as a vaccine against smallpox. However, other viruses, such as cowpox virus, may also be used. Several strains of vaccinia, which differ in level of virulence, are available for use as vaccine strains. For many purposes, a less virulent strain such as the New York City Board of Health (NYCBH) Strain is used.

## 2. Selective host cells and genes necessary for growth thereon

The selection system of the present invention is based upon the requirement of certain selective host cells that a virus contain a certain gene in order to replicate on the cell and form plaques. If this gene can be isolated and deleted from the viral genome using in vivo recombination or other mutagenesis techniques, then the virus will not be capable of growing on the host cell. Using this mechanism, viruses which lack the function of the gene necessary for replication on the host cell can be allowed to recombine with a plasmid vector which contains the gene necessary for replication. Only the recombinant virus will then grow on the selective host cell.

Recently, Gillard et al. Proc. Natl. Acad. Sci. USA, 83:5573 (1986) have mapped the gene which permits vaccinia virus replication on certain human cell lines to a gene which is located at the HindIII M,K junction and which encodes a 29 Kilodalton (KD) protein. Besides allowing replication on certain human cells, the presence of this gene enables vaccinia virus to replicate on RK13 (rabbit kidney) cells. Thus, the expression of the 29K gene is an absolute requirement for vaccinia virus replication on RK13 cells because this gene is necessary for translation of viral messages in these cells.

The requirement that a vaccinia virus have the 29K gene function for replication on selective host cells such as RK13 cells provides a basis for recombinant selection. Other eukaryotic host cells besides RK13 may be used. For example, a selection system based on Chinese Hamster Ovary (CHO) cells may be used in conjunction with a mutant virus, such as cowpox or vaccinia virus. Certain human cell lines, including KB, Hep$^2$, NCTC2544, Detroit 550, 809, HE$_1$, HE$_2$ and MRC$_5$ can also be used as non-permissive selective cell lines. Drillien et al., Virology, 111:488-499 (1981).

A mutant virus which is deleted for the 29K function can be created by creating a plasmid vector which contains DNA sequences homologous to pox viral genomic DNA sequences which flank the viral 29K gene. The vector contains a gene encoding a selectable marker (e.g., LacZ) located between the homologous DNA sequences. This vector is then recombined with a wild type virus. The recombination results in replacement of all or part of the 29K gene with the marker gene. Recombinant mutant viruses lacking 29K function can be selected for based on acquisition of the marker.

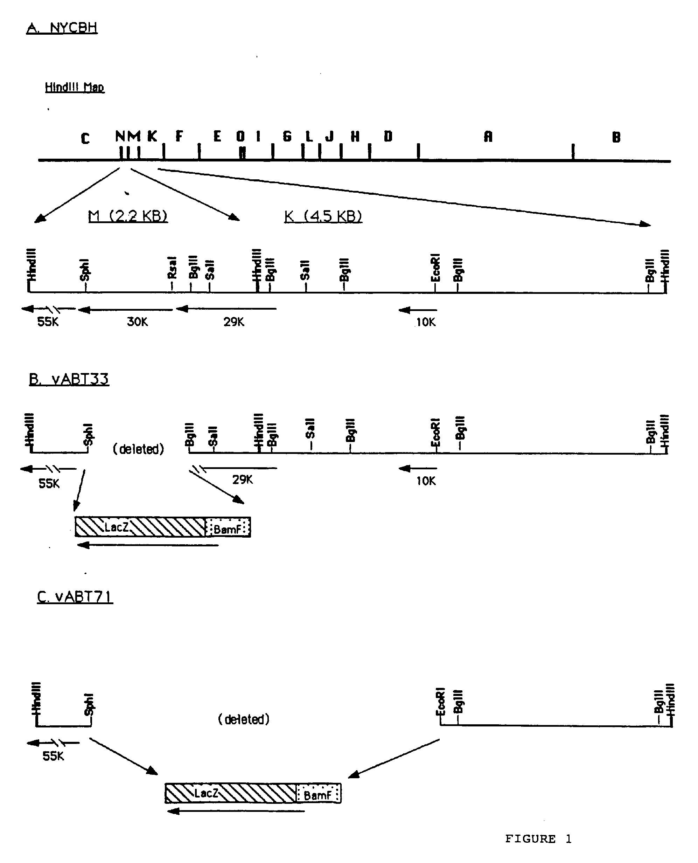

A mutant virus has been constructed, designated vABT33 (see Figure 1) using standard in vivo recombinant techniques, which is deleted for the 29K function and therefore, cannot replicate on RK13 cells. This mutant vABT33 virus is derived from the NYCBH wild-type strain, and forms plaques on BSC-40, CV1, and Hu143TK cells, yet does not replicate on RK13 cells. When the cloned 29K gene is allowed to recombine into this mutant, recombinant virus form plaques on RK13 cells. This technique has been successfully used to insert foreign genes along with the 29K gene at both the 29K gene location (HindIII M,K) and the TK location (HindIII J) of the vaccinia genome using donor DNA vectors. These recombinants form plaques on RK13 cells. The RK13$^+$ progeny are the desired recombinants, and are pure following plaque-purification on RK13 cells.

## 3. Donor DNA Vectors for Recombination with Pox Virus

According to the method of this invention, the gene necessary for the virus to replicate on a host cell is inserted into the genome of the virus so as to bestow the recombinant virus with the ability to replicate on the host cell. This is accomplished by first constructing a DNA donor vector for recombination with the virus. The vector contains the gene necessary for replication on the selective host cells and a foreign gene or genes, flanked by viral sequences and a viral promoter. The viral flanking sequences can be any DNA region which is non-essential for replication. These flanking sequences allow the donor vector to recombine with the virus in vivo at a specific region in the virus genome. This recombination results in integration of the foreign DNA and the gene necessary for replication on the selective host cells into the genome to produce a recombinant virus containing the foreign gene or genes, which is capable of replicating on selective host cells.

The donor DNA vectors of this invention for integration of a foreign gene into the viral genome contain the following elements:

a. a viral promoter

b. a foreign gene sequence

c. a DNA sequence containing the gene necessary to enable the virus to replicate on a host cell, and

d. DNA sequences flanking the construct of elements a-c, the flanking sequence being homologous to a region of the viral genome.

The viral promoter controls expression of the gene or genes and can be obtained from the species of virus with which the vector is designed to recombine.

For pox viruses, the sequences flanking the construct of elements a, b and c (a pox viral promoter, gene necessary for replication on the host cell and the foreign gene) are homologous to a region of the pox virus genome. Thus, recombination and integration of foreign DNA will occur at this site and the inserted DNA will not abolish viral replication.

In a preferred embodiment of this invention, the donor vector contains the 29K gene function to be inserted into the TK site; therefore, selection is by growing on RK13 cells. In order to obtain insertion into the TK site, the donor vector must contain flanking sequences homologous to the TK gene sequences.

A particularly preferred region for insertion into the pox virus genome is within the native site of the gene coding for the 29K function. The 29K gene has been shown to be necessary for the replication of vaccinia virus on RK13 cells because the product of this gene is essential for translation of viral messages on these cells. The 29K function is not essential for replication on non-selective cells, and recombinants can be selected by growing on RK13 cells.

Other regions of the pox virus genome can be used as flanking sequences to direct the stable integration of the DNA vector into the pox viral genome.

The preferred species of pox virus for insertion of foreign genes is the vaccinia virus. Accordingly, preferred vectors are designed for recombination with the vaccinia virus and thus, the pox viral elements of the vector are derived from vaccinia virus. A preferred vector for recombination with vaccinia virus may contain

a. a vaccinia promoter sequence (e.g., the vaccinia 11K, 7.5K or 30K promoter or derivatives thereof),

b. a foreign gene or genes,

c. a second vaccinia promoter,

d. a DNA sequence necessary for replication on a host cell,

e. DNA flanking sequences homologous to a region of the vaccinia virus the DNA sequences flanking the construct of elements a-d (e.g., sequences of the vaccinia TK gene).

Vaccinia promoters are DNA sequences which direct messenger RNA synthesis from vaccinia gene during a vaccinia virus infection. Such promoters can be isolated from the vaccinia genome or can be constructed by DNA synthesis techniques.

## 4. Genes for Integration into Pox Virus

Foreign genes for integration into the genome of a virus in expressible form can be obtained by any conventional technique for isolating a desired gene. The genes can be derived from organisms including bacteria, viruses or other microorganisms. In many cases, those foreign genes are antigenic determinants suitable for use in a virus-based vaccine.

For organisms which contain a DNA genome, the genes encoding an antigen of interest are isolated from the genomic DNA or cDNA; for organisms with RNA genomes, the desired gene may be isolated from cDNA copies of the genome. If restriction maps are available, strategies can be designed for cleaving genomic DNA by restriction endonuclease digestion to yield DNA fragments that contain the gene of interest. In some cases, desired genes may have been previously cloned and the genes can be obtained from available clones. Alternatively, if the DNA sequence of the gene is known, the gene can be synthesized by any of the conventional techniques for synthesis of deoxyribonucleic acids (e.g., the phosphate or phosphite triester techniques).

Genes encoding an antigen of interest can be prepared for insertion into the DNA vectors designed for recombination with virus by standard techniques. In general, the cloned genes can be excised from the prokaryotic cloning vector by restriction enzyme digestion. In some cases, the excised fragment will contain the entire encoding region of the gene, including its translational start signal; in others, it will be absent. The DNA fragment carrying the cloned gene can be modified as needed, for example, to make the ends of the fragment compatible with the insertion sites of the DNA vectors used for recombination with virus, then purified prior to insertion into those vectors at restriction endonuclease cleavage sites.

## 5. In Vivo Recombination

The donor plasmid vectors containing the gene necessary for replication on a selective host cell (the "host-replication" gene), and the foreign gene, flanked by appropriate viral sequences, will undergo recombination with virus genomic DNA resulting in integration of the flanked genes into the viral genome. Recombination occurs in a eukaryotic host cell. Appropriate host cells for recombination are those which can be infected by virus and transfected by the donor vector. Examples of such cells are CV-1 (monkey kidney cells), Hu143TK (human cells), BSC40 (monkey kidney cells), RK13 (rabbit kidney) cells.

Viral infection is accomplished by standard techniques for infection of eukaryotic cells with virus. Cells are co-transfected with the donor plasmid by any of the conventional techniques of transfection. After infection and transfection, the cells are incubated under standard conditions and the virus is allowed to replicate, during which in vivo recombination occurs between the homologous viral sequences in the donor vector and the viral sequences in the genome.

## 6. Selection Step

Recombinant progeny are then selected by plating and plaque-purifying on selective host cells. Virus which have recombined with the donor plasmid will have in their genomic DNA the gene necessary for replication on these host cells. The starting unrecombined virus will lack the host-replication gene and will not grow on the selective host cells.

A preferred embodiment of this invention utilizes the vaccinia virus 29K gene as the selectable marker in the construction of vaccinia virus recombinants. Since this gene is required for replication on RK13 cells, any recombinants which express this gene will have a powerful selective advantage over non-recombinant virus which are deleted for this gene, when plaque purified on RK13 or other selective host cells. This approach can be used to select for recombinant virus which contain the 29K gene. Recombinants are selected for by their ability to form plaques on RK13 cells. Using this techniques, foreign genes inserted with the host-replication gene will also be expressed by the recombinants.

This technique has been used to insert the Hepatitis B surface-antigen, for example, into the viral genome of a mutant vaccinia virus, which lacks the 29K gene. The gene encoding the antigen, under the transcriptional control of a promoter (e.g., the 11K or 30K promoter) was inserted into the viral 29K native site along with the 29K gene. Recombinants were screened using an radioimmunoassay specific for the antigen, and for their ability to form plaques on selective host cells, such as RK13 cells. After the second round of plaque purification, the isolates were believed to be pure based on the quantitiative expression of the s-antigen and by the viral titers under both selective and non-selective conditions.

The 29K gene was also used as a basis for selection for recombinants inserted into the TK gene. The pseudorabies glycoprotein 50 (gp 50) gene, under the transcriptional control of a promoter the (e.g., the 7.5K promoter), was inserted into the TK gene along with the 29K gene. Recombinants were screened using a black plaque assay, BUDR resistance (which selects for the TK⁻ phenotype), and for their ability to form plaques on selective cells, such as RK13 cells. At all stages of the plaque-purification, gp50 was quantitatively expressed on both RK13 cells and Hu143TK⁻ cells in the presence of BUDR. The relative titers of individual plaque-picks was compared for both selective (RK13 or TK⁻) and non-selective conditions (Hu143TK⁻). After the second round of plaque purification the isolates were judged to be pure on the basis of the quantitative expression of gp50 under both non-selective and selective conditions and by the fact that the viral titers were equivalent for both selective and non-selective conditions. Southern blot analysis confirmed the structures of the isolates and the black plaque assay confirmed the expression of the inserted antigen.

This invention shows that the 29K/RK13 selection system is as powerful as the thymidine kinase selection system. The selection system of the invention, however, does not require the use of drugs, mutagens, chromogenic indicators or foreign genes and is not restricted to insertion of the desired antigen at the 29K native genomic location. Because the 29K selection system is a positive selection system which requires the insertion of the 29K gene, duplicated structures, which can form from a single recombination event, for example the negative TK selection (TK⁻/BUDR), cannot be eliminated. However, these duplicated structures are unstable, and rapidly degenerate in vaccinia virus; therefore so two rounds of plaque purification are usually sufficient to remove these intermediates from the population. The use of RK13 cells for the amplification of viral plaque-picks further insures the purity of the resulting viral stocks.

This recombinant selection scheme will be great utility to those involved in recombination studies, in the construction of expression vectors and, most important, for the construction of vaccinia live viral recombinant vaccines where the use of unnecessary foreign genes, drugs, mutagens or chromogenic indicators is prohibited.

This technique has also been used to insert simian viral antigens into vaccinia virus and to select recombinant virus using RK13 cells.

The invention is further illustrated by the following Exemplification.

## EXEMPLIFICATION

## Materials and Methods

## Cell Lines and Viruses

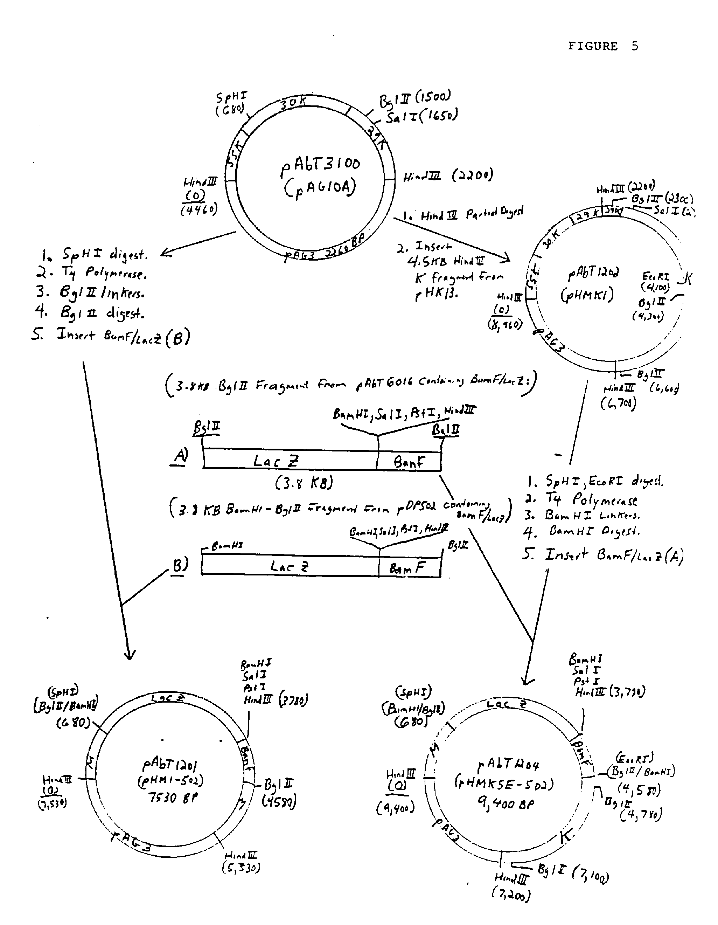

The human TK⁻ 143 cell line (Hu143TK⁻), monkey kidney cell line Bsc-40, CV1 and the rabbit kidney cell line RK13 (Beale et al., (1963) Lancet, 2, 640) were used in this work. The 29K deficient virus vABT33 (Applied Biotechnology, Inc.) contains a deletion in HindIII M from SphI to Bg1 II which removes 93 nucleotides (BP) of coding sequence from the 3' end of the 29K gene. Inserted into this deletion is the HindIII F weak early promoter (D. Panicali et al., (1986) Gene, 47:193-199) upstream from the beta- galactosidase gene colinear with the transcriptional organization of this region of the genome J.R. Morgan and B.E. Roberts (1984), J. Virol., 51:283-287. (see Figure 1). This recombinant was produced by in vivo recombination between the vector pAbT1201 (the construction of which is shown in Figure 5) and the New York City Department of Health (NYCBH, Wyeth Laboratories, ATCC # VR-325) vaccine strain, and forms plaques on Bsc-40, CV1 and Hu143TK⁻ cells, but does not replicate on RK13 cells.

## Donor DNA Vectors

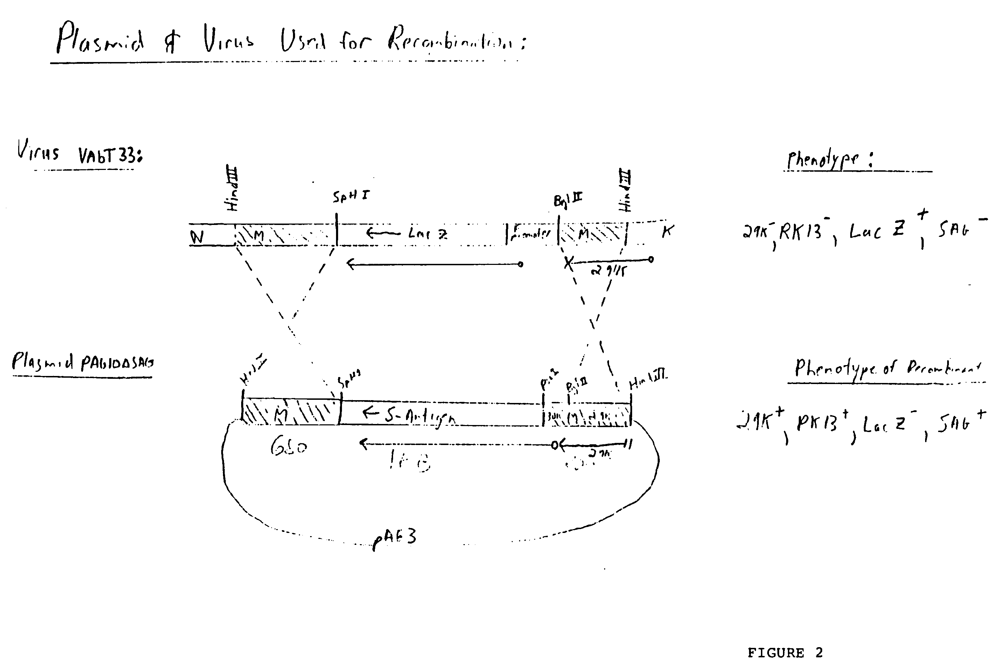

Plasmid pAG10SAG is deleted from Sph I to Rsa I in HindIII M as shown in Figure 2. It contains the 3' end of the 29K gene from the right HindIII site of HindIII M through the termination codon and the 5' promoter element the 30K promoter (Perkus et al., (1985) Science, 229:881) of the next gene upstream of the Hepatitis B s-antigen gene (see Figure 2). J.R. Morgan and B.E. Roberts, (1984), J. Virol., 51:283-297. Thus, upon recombination with vABT33, this plasmid will restore the 3' end of the 29K gene and insert the Hepatitis B s-antigen gene under the transcriptional control of the 30K promoter.

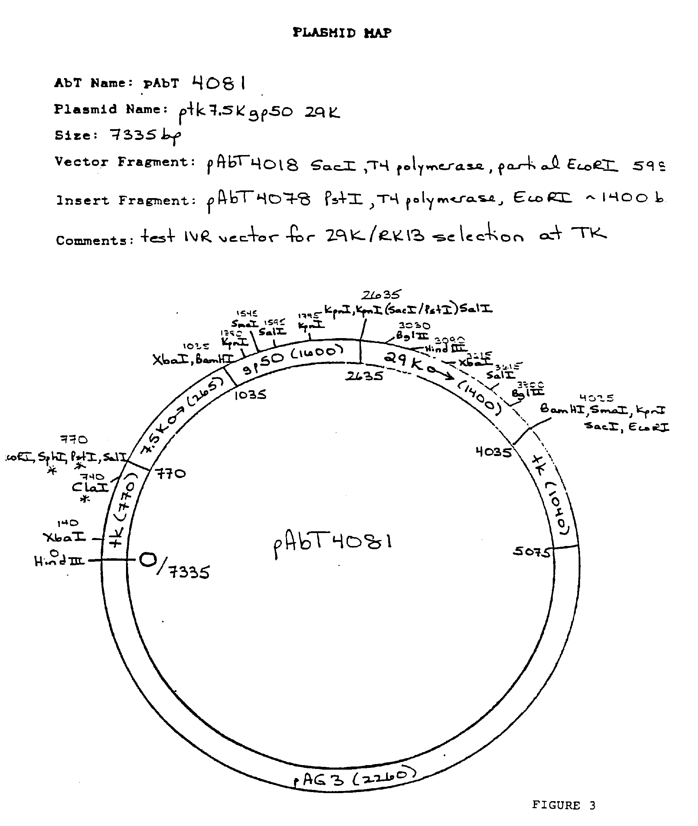

Plasmid pTK29K7.5Kgp50 contains the vaccinia virus 7.5K promoter upstream from the pseudorabies glycoprotein 50 gene in tandem with the Sa1 I to Rsa I fragment from the HindIII M,K junction which contains the 29K gene in its entirety as shown in Figure 3. These genes are inserted into the EcoRI site of the TK gene located in the HindIII to PvuII fragment of the vaccinia virus HindIII J fragment. Thus, recombination of this plasmid into the TK gene of vABT33 virus will result in a recombinant virus with the following phenotype: TK⁻, gp50⁺, 29K⁺, LacZ⁺, (see Figure 3).

## In vivo Recombinations, Plaque Assays and Plaque Purifications

In vivo recombinations were performed on non-selective cells (either Bsc-40 or CV1) using mutant virus vABT33 and 20 ug of the appropriate plasmid DNA as previously described. (D.D. Spyropolous et al., 1988, J. Virol., 62:1046-1054). Plaque assays were performed on both non-selective (CV1, Bsc-40 or HU143TK⁻) and selective cells (RK13, HU143TK⁻/BUDR) in duplicate. 5-bromodeoxyuridine (BUDR) was used to select TK⁻ recombinants at 60 ug/ml. After 48 hours of plaque formation under a nutrient agar overlay, monolayers were subjected to a black-plaque assay or overlayed with a second agar overlay containing 400 ug/ml of Bluo-Gal (halogenated indolyl-B-D-galactoside, BRL Inc.) and evaluated for beta-galactosidase expression after 48 hours of further incubation. Individual plaques were picked from agar overlays using a pasteur pipette, and virus-containing agar plugs were freeze/thawed three times before being titered on both selective and non-selective cells. There were subsequently assayed for antigenic expression before being picked and retitered.

## Black Plaque Assays

Viral plaques were assayed for the production of pseudorabies glycoprotein 50 throughout the plaque-purification of recombinant virus vTK29K7.5Kgp50 using a black plaque assay as described by Mackett et al. in EMBO, 4:3229-3234 (1985). One half of the duplicate titrations of each round of plaque-purification was subject to this assay while the other half was used for picking individual plaques from RK13 cells for further purification. After 48 hours of plaque formation under agar medium the agar overlays were removed and the infected monolayers were processed as follows. Dishes were washed three times with 1 ml of phosphate buffered saline (PBS) and fixed using 1 ml of 3.7% formaldehyde in PBS for 30 minutes at ambient temperature. After three more PBS washes the fixed monolayers were treated with 1 ml of a 1:1000 dilution of mouse anti-gp50 monoclonal antibody (MCA 50; a gift from Rebecca Hyde, National Veterinary Services Laboratory, Ames, Iowa) dissolved in 50% nonimmune goat sera (NGS) in PBS. After a one hour incubation at room temperature the dishes were washed three times with PBS and treated with 1 ml of a 1:1000 dilution of alkaline phosphatase conjugated goat anti-mouse IgG dissolved in 10% NGS in PBS for one hour at room temperature. The plates were then washed twice with one ml of PBS and once with one ml of TBS (20 mM Tris pH 7.6, 150 mM NaCl). Antibody binding was visualized using the phosphate substrate system (Kirkegaard & Perry Labs, Inc.) according to manufacturers suggestions. After 30 minutes of color development plates were counted for the number of gp50 positive plaques.

## Radioimmunoassay for Hepatitus B s-Antigen

After the second round of plaque-purification of recombinant virus v30KSAG, plaques which formed on RK13 cells were picked and resuspended in 2 ml of DMEM/2% FCS and subject to three freeze/thaw cycles.

100 ul of each resuspended plaque was used to infect $10^6$ confluent non-selective (Bsc40) cells. Dishes were freeze/thawed three times after total cytopathic effect was observed (48 hours) and their contents (5 ml) transferred to tubes. Two hundred (200) ul of each extract was used as a source of antigen in the AUSRIA III RIA for hepatitus B s-antigen kit (Abbott Laboratories) and the assay was performed according to the manufacturers instructions.

Genomic Structual Analysis of Recombinants

After the second round of plaque purification of recombinants, individual plaques were picked and resuspended in 2 ml of DMEM, 2% FCS. One hundred (100) ul of each plaque-pick was used to infect $10^6$ non-selective (Bsc40) cells. After 48 hours of infection, vaccinia virus genomic DNA was prepared by the method described by Campione-Piccardo et al. in J. Virol. 31:281-287 (1979). Viral DNA was then digested with the appropriate restriction enzyme and subjected to southern blot analysis. E.M. Southern, J. Mol. Biol., 98:503-517 (1975)

Example 1

Mutant virus vABT33 Does Not Replicate on RK13 Cells

Vaccinia virus mutant vABT33 was prepared using the method set forth above, and plaqued onto RK13, HU143TK⁻, CV1 and Bsc40 cells. Wild-type (NYCBH) vaccinia virus was used as a control. Mutant virus vABT33 produced similar yields of virus on Hu143TK⁻, CV1 and Bsc40 cells as wild-type (NYCBH) virus; yet on RK13 cells, there was a $10^5$ decrease in viral yields, as shown on Table 1.

## Table 1

### Comparison of viral yield of wild-type (NYCBH) and 29K deleted mutant (ABT33) on permissive and non-permissive cell lines for the 29K deleted virus

| | NYCBH | ABT33 |
|---|---|---|
| Hu143TK⁻ | $5 \times 10^7$ PFU | $9 \times 10^7$ PFU |
| RK13 | $1 \times 10^9$ PFU | $2 \times 10^4$ PFU |

PFU= plaque forming units

Furthermore, no plaques were formed on RK13 cells when virus was harvested from a control in vivo recombination without the addition of a 29K rescuing plasmid. Therefore, a functional 29K gene is absolutely necessary for viral replication on RK13 cells and provides a powerful selective advantage to recombinants containing this gene over vABT33 when titered on RK13 cells. The results comparing the control and recombinant virus on CV1 and RK13 cells are shown on Table 2:

### TABLE 2

Initial Titration curve of the viral recombination products between ABT33 virus and plasmid PAG10SAG on CV1 and RK13 cells

| | ABT33 ALONE | | ABT33+PAG10SAG | |
|---|---|---|---|---|
| | CV1 | RK13 | CV1 | RK13 |
| $10^{-1}$ | TMTC[1] | 0* | TMTC | 0* |
| $10^{-2}$ | " | " | " | " |
| $10^{-3}$ | " | NORMAL | " | 8/10 |
| $10^{-4}$ | " | " | " | 1/2 |
| $10^{-5}$ | 44/53 | " | 59/71 | 0 |

\* Monolayers had no plaques, but expressed beta-galactosidase and eventually died.

[1]TMTC= To Many To Count.

The percentage of recombinants is calculated by dividing the number of plaque forming units (PFU) on the RK13 cells by the total number of PFU:

$$\% \text{ Recombinants} = \frac{1.8 \times 10^4 \text{ PFU on RK13}}{1.3 \times 10^7 \text{ total PFU}} = 0.14\% \text{ recombinants}$$

Example 2

Insertion of Hepatitis B s-antigen gene into mutant vaccinia virus using the 29K gene

PAG10SAG Plasmid Recombinants

Mutant vaccinia virus vABT33 was transfected with the pAGlOSAG donor plasmid. Plasmid pAglOSAG was designed to insert the 29K gene followed by the 30K promoter driving Hepatitis B s-antigen into the 29K native site of the viral genome. The recombinant virus was designated v30KSAG. The relative purity of recombinants inserted into the 29K region of vABT33 was assayed at every stage of the plaque-purification of v-30KSAG virus by examining beta-galactosidase expression of viral plaques using Bluo-Gal in the agar overlay. Stable recombinants did not contain the LacZ gene and therefore, formed clear plaques on both selective and non-selective cells; while vABT33 formed blue plaques only on non-selective cells. Table 2 shows that the pAG10SAG plasmid, upon recombination with vABT33, restores the 29K gene in its entirety, and yields viral progeny which will form plaques on RK13 cells. The yield of recombinate RK13[+] progeny (0.14% of total virus) is within the range of the yield of vaccinia virus recombinants in a normal in vivo recombination. No plaques were formed on RK13 cells in a duplicate in vivo recombination using vABT33 without the addition of rescuing DNA; yet cytopathic effects and beta-galactosidase expression were apparent at high multiplicities of infection ($10^{-1}$ - $10^{-2}$ dilution; MOI = 0.1 -1 PFU/cell) for both the minus-DNA control and the in vivo recombinant itself. After 5 days of incubation, approximately one half of the RK13[+] plaques expressed beta-galactosidase and formed blue plaques in the presence of Bluo-gal. Both blue and colorless plaques were picked and replated on selective and non-selective cells. The results are shown on Table 3.

## Table 3

Titration of first-round plaque-picks of PAG10SAG in vivo recombinants on non-selective (CV1) and selective (RK13) cell lines in the presence of Bluo-gal.

| Plaque # | # Blue/# White[1] | | | |
|---|---|---|---|---|
| | CV1 Cells | | RK13 Cells | |
| | | %White | | %White |
| 1* | 3/14 | 82 | 9/11 | 55 |
| 2* | 0/1 | 100 | 0/0 | --- |
| 3* | 6/28 | 82 | 6/23 | 79 |
| 4* | 19/8 | 30 | 6/22 | 79 |
| 5 | 0/120 | 100 | 0/230 | 100 |
| 6 | 0/300 | 100 | 10/340 | 97 |
| 7 | 9/30 | 77 | 10/120 | 92 |
| 8 | 0/2 | 100 | 0/0 | --- |
| 9 | 0/200 | 100 | 0/440 | 100 |

* Plaques 1-4 were deliberately picked and plated as "blue" plaques on RK13 cells from an initial in vivo recombination.

[1] Values represent PFU per 300 ul from 2 ml of an individual plaque-pick.

Table 4 illustrates the purity of recombinant plaques formed on RK13 cells after two rounds of plaque purification. Plaque-picks which have been plated on both selective and non-selective cells are almost pure; even "blue plaques" from the in vivo recombination are 50% pure, while some colorless plaque picks yield 100% apparent recombinants, thus demonstrating the power of the 29K/RK13 selection system.

## Table 4

### Titration of second round plaque-picks of PAG10SAG in vivo recombinants on non-selective (CV1) and selective (RK13) cell lines

#### Percent White Plaques

| Plaque # | CV1 Cells | RK13 Cells |
|---|---|---|
| 1 | 100 | 100 |
| 2 | 100 | 100 |
| 3 | 100 | 100 |
| 4 | 100 | 100 |
| 5 | 100 | 100 |
| 6 | 100 | 100 |
| 7* | 95% | 95% |
| 8* | 95% | 95% |
| 9* | 95% | 95% |
| 10* | 95% | 95% |

* Plaques 7-10 were deliberately pickled as blue plaques from the first round titration.

The titration of the initial in vivo recombination on RK13 cells provided a greater than 600 fold purification of white plaque recombinants in one step. At this round of plaque-purification there were no beta-galactosidase expressing plaques derived from colorless plaques from the previous round; these isolates were pure desired recombinants.

Example 3

Insertion of pseudorabies glycoprotein-50 antigen gene using the 29K into the mutant vaccinia gene

pTK29K7.5Kgp50 Plasmid Recombinants

Mutant virus vABT33 was transfected with the pTK29K7.5Kgp50 donor plasmid using methods previously described herein. The recombinant virus was designated vTK29K7.5Kgp50. Plasmid pTK29K7.5Kgp50 was designed to insert the 29K gene followed by the 7.5K promoter driving pseudorabies virus glycoprotein 50(gp50) into the TK gene in the viral genome, thereby interrupting it. Thus the desired recombinant virus vTK29K7.5Kgp50, will have the following phenotype: 29K+, gp50+, TK⁻. During the plaque pick of this recombinant all isolates were plated out in duplicate on selective RK13 and non-selective Hu143TK⁻+/-BUDR.

Table 5 demonstrates that 92% of the RK13⁻ plaques derived from the in vivo recombination express gp50, compared to 0.86% gp50 expressing recombinants on non-selective Hu143TK⁻ cells. The same extracts assayed on Hu143TK⁻ cells with BUDR yielded a strikingly similar amount of gp50 expressing recombinant (3.6 x 10⁵ PFU on TK⁻ versus 3.6 x 10⁵ PFU on RK13 cells) demonstrating the similar power of selection for both selection schemes. Plaques were picked from a parallel RK13 plate which had not been processed for the black plaque assay.

## Table 5

### Initial In Vivo Recombination
### of v29K7.5Kgp50 plasmid

# Black/Total

| Cell Type: | TK⁻ | RK13 | TK⁻/BUDR |
|---|---|---|---|
| $10^{-2}$ | - | - | - |
| $10^{-3}$ | - | TMTC | TMTC |
| $10^{-4}$ | TMTC | 36/39 | 26/40 |
| $10^{-5}$ | 6/500 | - | - |
| $10^{-6}$ | - | - | - |
| Total PFU: | $5.1 \times 10^{7}$ | $3.9 \times 10^{5}$ | $4 \times 10^{5}$ |
| Black PFU: | $4.4 \times 10^{5}$ | $3.6 \times 10^{5}$ | $3.6 \times 10^{5}$ |

As in the first in vivo recombination experiment, picked plaques were plated in duplicate on both selective (RK13, Hu143TK-/BUDR) and non-selective (Hu143TK⁻) cells.

Six isolates picked from RK13 cells were plated on RK13 cells, Hu143TK cells and Hu143TK⁻ cells with BUDR. All isolates formed plaques on RK13 cells, and approximately 80% of these plaques expressed gp50. As expected, almost all of the plaques which formed on TK⁻ cells express gp50. There are, however, less plaques on the BUDR plate than the TK⁻ plate without the drug. This suggests that there is still a significant amount of TK⁺ virus in the plaque picks at this stage in the plaque-purification. These TK⁺ varions may be non-recombinants or single recombinants containing duplicated TK sequences from both the virus and recombinant plasmid. Because RK13 selection cannot eliminate duplicated structures as BUDR can in this case, these unstable recombinants may still be present in this population. Again, plaques were randomly picked from a duplicate RK13 plate and retitered on RK13, 143HUTK⁻ and 143HUTK⁻/BUDR cells.

All retitered plaque-picks yielded 100% plaques that express gp50 on all cells and selections used; therefore, these plaques were shown to be pure at this stage of the plaque-purification. The consistent titers between cell lines and selections used confirms the purity of the recombinants.

Example 4

Genomic Structural Analysis

A. v30KSAG Recombinant Virus

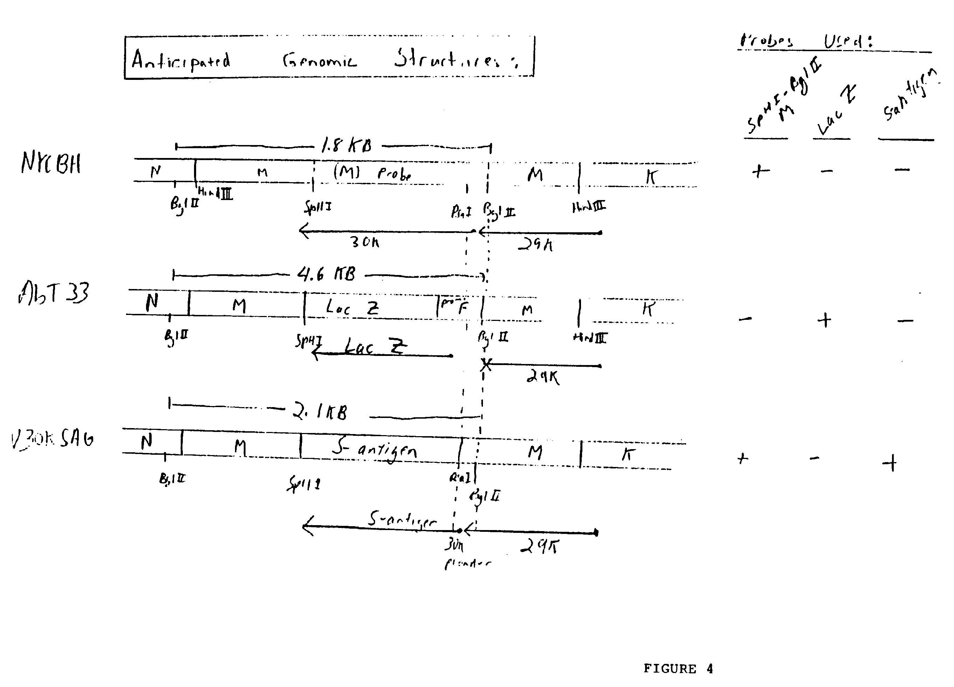

Figure 4 illustrates the predicted genomic structure of wild-type NYCBH virus, and of recombinant viruses vABT33 and v30KSAG. Three probes were used to characterize these recombinants: (1) the 820 base pair(bp) SphI to Bg1II fragment from HindIII M which contains the entire sequence deleted from vABT33, the 3' end of the 29K gene and 5' promoter element that is restored in v30KSAG and used to promote transcription of the Hepatitus B s-antigen gene (SAG); (2) the 3.1 kb LacZ fragment which should only be present in vABT33; and (3) the SAG gene which should be present in v30KSAG recombinants. A Bg1II digest was performed on all genomic DNAs in order to provide a specific size fragment containing the sequences described above.

All of the v30KSAG isolates contained SAG hybridizable sequences which were located within a 2.1 kb Bg1II fragment as predicted. The 820 bp HindIII M probe demonstrated the deletion of these sequences in vABT33, the size of the corresponding BG1II fragment in NYCBH virus, and that all v30KSAG candidates contain the 3' end of the 29K gene and the 5' end of the 30K gene. It also showed that this BglII fragment is of the correct size in these recombinants (2.1 kb). The LacZ probe confirmed the structure of vABT33; it hybridized with a 4.6 kb Bg1II fragment as predicted. Two white v30KSAG candidates did not contain LacZ sequences as predicted, yet the three "blue" isolates which were amplified on non-selective Bsc40 cells contain trace amounts of LacZ sequences at the same size (4.6 kb) as vABT33 virus.

Southern blot analysis confirmed the structure of the recombinants and elucidated the structure of the infrequent LacZ[+], RK13[+] plaques.

## vTK29K7.5Kgp50 Recombinant Virus

In order to characterize the structure of these recombinants, the following probes were used: (1) The 29K fragment which should demonstrated the insertion of the 29K gene into these recombinants, and (2) The Sa1I to SA1I gp50 fragment which demonstrated the insertion of the gp50 gene into TK.

Southern blot analysis demonstrates that the structures are as predicted.

## EXAMPLE 5

## Antigenic Expression Assays

## Black Plaque Assay for gp50

Black Plaque assays were performed as described herein. It was found that recombinants from second round amplification on Bsc40 cells express gp50 and therefore are plaque-purified to homogeneity.

## RIA for Hepatitis B S-Antigen

A commercial radioimmunoassay kit AUSRIA III RIA (Abott Laboratories) was used to detect the presence of Hepatitis B surface antigen. Amplification of both LacZ[−] and LacZ[+] plaques which formed on RK13 cells expressed s-antigen. Thus, the 29K selection yielded recombinants which contained the desired insertion and expressed the desired antigens which is consistent with the Southern blot analysis results for this experiment.

## EXAMPLE 6

## Construction of recombinant vaccinia viruses containing SIVMAC-251 genes

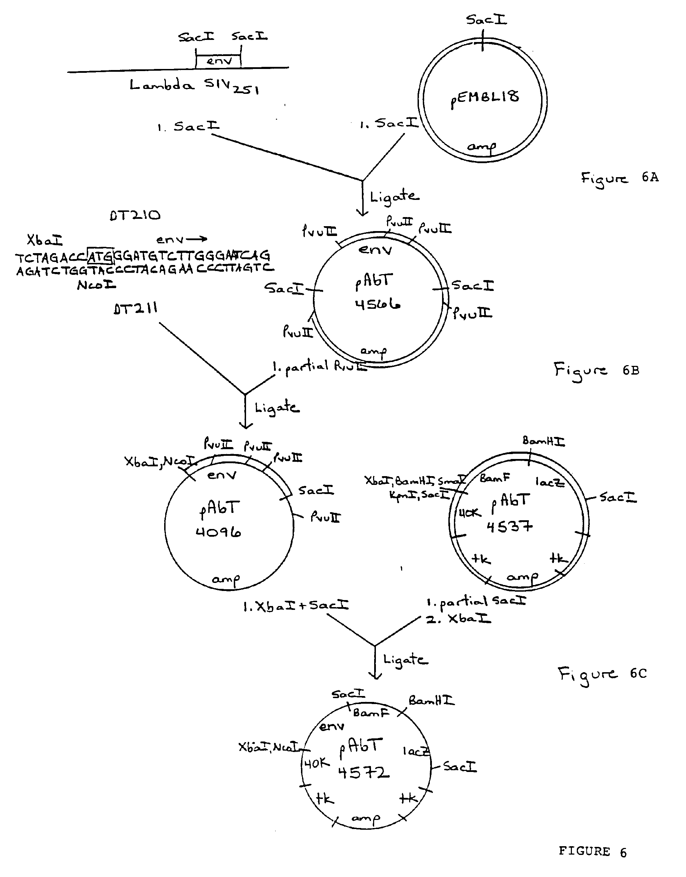

Bacteriophage lambda EMBL4 containing full-length proviral SIV macaque strain 251 (SIV$_{MAC-251}$) DNA was obtained from Ronald C. Desrosiers (New England Regional Primate Research Center (NERPRC), Southborough, MA). This DNA, denoted lambda SIV$_{251}$, was digested with SacI and a 3500bp fragment containing the gp160-encoding region (env) was gel-purified. pEMBL18 (Dente et al., (1983) Nucl. Acids Res. 11:1645) was digested with SacI and was ligated to the 3500bp fragment to create pAbT4566, as shown in Figure 6.

To insert a convenient restriction site 5' to the initiation codon ATG of the SIV$_{MAC-251}$ env gene, two complementary oligonucleotides, DT210 and DT211, were synthesized (Dept. of Biology, Brandeis University); their sequences are shown in Figure 6B. pAbT4566 was partially digested with PvuII, and a 6500bp fragment containing all but the 5' 21bp of env and the 5' flanking region of env was gel-purified and ligated to DT210 and DT211 to create pAbT4096, as shown in Figure 6B. The env gene in pAbT4096 now has an XbaI site and an NcoI site 7 and 1bp, respectively, 5' to the env initiation codon ATG.

pAbT4096 was digested with XbaI and SacI, and a 2600bp fragment was gel-purified. pAbT4537 was partially digested with SacI and was completely digested with XbaI. The resulting 7300bp fragment was gel-purified and ligated to the 2600bp fragment to create pAbT4572, as shown in Figure 6C.

pAbT4572 is a vector for the insertion and expression of SIV$_{MAC-251}$ env in vaccinia. pAbT4572 contains the env gene under the control of the vaccinia 40K promoter, the DNA regions flanking the vaccinia TK gene for directing recombination in vaccinia, the lacZ gene under the control of the vaccinia BamF promoter for selection of vaccinia recombinants and a bacterial replicon and ampicillin-resistance gene for growth and selection E. coli (Figure 6C).

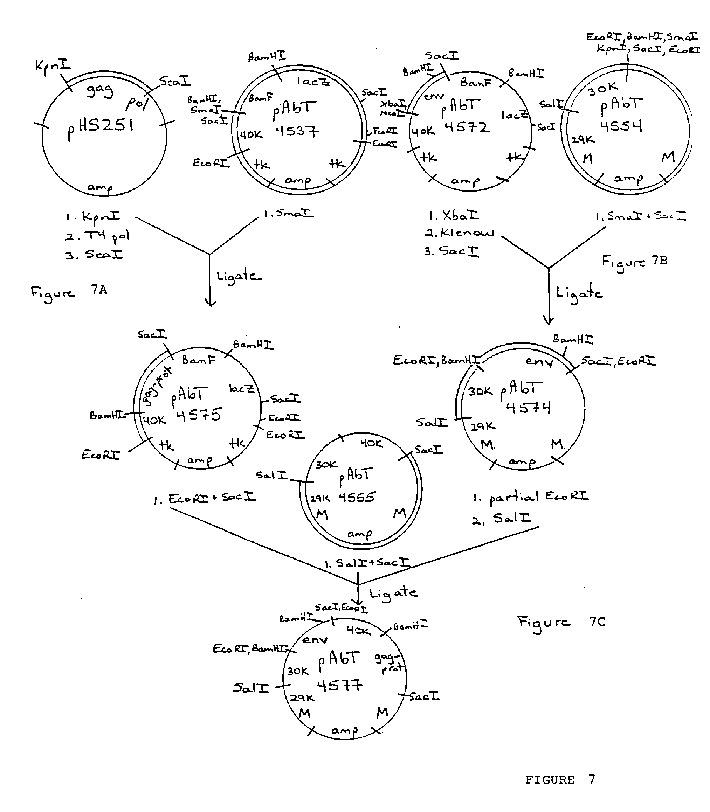

Plasmid pHS251, containing the central portion of the SIV$_{MAC-251}$ proviral genome, was obtained from Ronald C. Desrosiers (NERPRC). This DNA was digested with KpnI, treated with T4 DNA polymerase, then was digested with ScaI, and a 2200bp fragment containing the gag gene was purified. This fragment also contains the 900bp of the gene encoding the pol polyprotein, including the entire coding sequence of the protease required for the processing of the gag polyprotein; this fragment is therefore denoted as containing the gag-prot gene. pAbT4537 was digested with SmaI and was ligated to the 2200bp fragment to create pAbT4575 as shown in Figure 7A.

pAbT4575 is a vector for the insertion and expression of SIV$_{MAC-251}$ gag and protease in vaccinia. pAbT4575 is identical to pAbT4572 described above, except that pAbT4575 contains the gag-prot gene under the control of the vaccinia 40K promoter (Figure 7A).

pAbT4572 was digested with XbaI, treated with Klenow, then digested with SacI, and a 2600bp fragment containing the env gene was gel-purified. pAbT4554 was digested with SmaI and SacI, and was ligated to the 2600bp fragment to create pAbT4574, as shown in Figure 7B.

pAbT4574 was partially digested with EcoRI and was completely digested with Sa1I, and a 3000bp fragment containing the 30K promoter and env gene was gel-purified. pAbT4575 was digested with EcoRI and SacI, and a 2400bp fragment containing the 40K promoter and the gag-prot gene was gel-purified. pAbT4555 was digested with Sa1I and SacI, and a 3500bp fragment was gel-purified and ligated to the 3000bp and 2400bp fragments to create pAbT4577, as shown in Figure 7C.

pAbT4577 is a vector for the insertion and expression of SIV$_{MAC-251}$ env and gag-prot in vaccinia. pAbT4577 contains the env gene under the control of the vaccinia 30K promoter and the gag-prot gene under the control of the vaccinia 40K promoter, flanked by vaccinia DNA for directing recombination into the vaccinia HindIII M region. The vector DNA includes the 29K host-range gene for selection of vaccinia recombinants and a bacterial replicon and ampicillin-resistance gene for growth and selection in E. coli (Figure 7C).

The vector pAbT4577, was transfected into BSC-40 cells which had been infected with vaccinia virus vAbT33. Recombinant viruses were selected as described above as white plaques in the presence of Bluogal on RK13 cells. Plaques were picked and purified, and were shown, by Southern analysis, to contain the appropriate SIV$_{MAC-251}$ gene(s): vAbT198 contains env & gag-prot.

The purity of these recombinants were monitored throughout the plaque-purification process by testing for the absence of beta-galactosidase expression for the v30KSAG virus; or the expression of pseudorabies gp50 antigen by individual recombinant plaques using a black plaque assay for'the vTK29K7.5Kgp50 virus.

## EXAMPLE 7 Construction of a divalent in vivo recombination vector

The precursor vectors were prepared as described in U.S. patent application serial no. 205,454, entitled, "A Method of Evaluating Genetically Enginneered Recombinant Vaccines Against Human Immunodeficiency Virus Using Animal Model Systems", attorney's docket No. ABT88-04, filed concurrently herewith, the teachings of which are incorporated by reference herein.

This vector contains the SIV$_{MAC-251}$ env gene under the control of the vaccinia 40K promoter and the SIV$_{MAC-251}$ gag-prot gene under the control of the vaccinia 7.5K promoter, with insertion at the vaccinia HindIII M region.

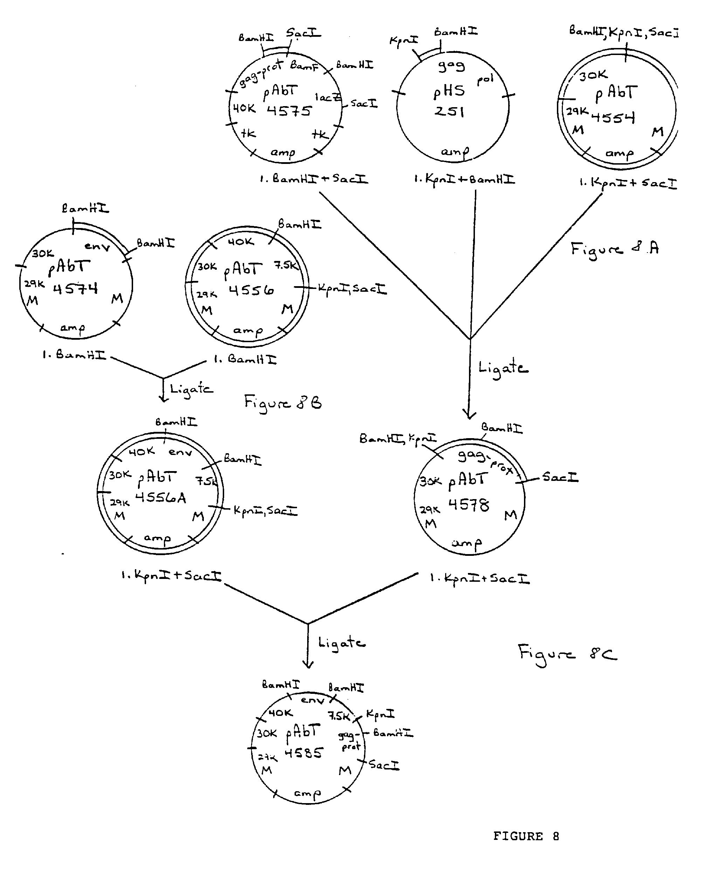

pAbT4575 was digested with BamHI and SacI, and a 1300bp fragment containing the 3' portion of the gag-prot gene was gel-purified. pHS251 was digested with KpnI and BamHI, and a 900bp fragment containing the 5' portion of the gag gene was gel-purified. pAbT4554 was digested with KpnI and SacI, and was ligated to the 1300bp and 900bp fragments to create pAbT4578, as shown in Figure 8A.

pAbT4574 was digested with BamHI, and a 2600bp fragment containing the env gene was gel-purified. pAbT4556 was digested with BamHI and was ligated to the 2600bp fragment to create pAbT4556A, as shown in Figure 8B.

pAbT4578 was digested with KpnI and SacI, and a 2600bp fragment containing the gag-prot gene was gel-purified. pAbT4556A was digested with KpnI and SacI, and was ligated to the 2600bp fragment to create pAbT4585, as shown in Figure 8C.

pAbT4585 is a vector for the insertion and expression of the SIV$_{MAC-251}$ env and gag-prot genes in vaccinia. pAbT4585 is identical to pAbT4577, except that pAbT4585 contains the env gene under the control of the 40K promoter and the gag-prot gene under the control of the 40K promoter and the gag-prot gene under the control of the 7.5K promoter (Figure 8C).

Equivalents

The present invention may be embodied in other specific forms without departing from spirit and scope thereof. These and other modifications of the invention will occur to those skilled in the art and intended to fall within the scope of the appended claims.

## Claims

1. A method for selecting for recombinant pox viruses, comprising the steps of:
   a. recombining a pox virus which lacks the function of the 29K gene or portion thereof required for replication of the pox virus in selective host cells for which the 29K gene is required for replication, and a donor DNA vector, the donor DNA vector comprising (i) a DNA sequence encoding a foreign gene, (ii) pox virus DNA sequences containing the 29K gene or portion thereof sufficient to restore 29K function in the pox virus, and (iii) flanking pox viral DNA sequences homologous to a region of the pox viral genome; and
   b. selecting for recombinant pox viruses based on the ability of the virus to replicate on the host cells.

2. A method of selecting for recombinant pox viruses which comprises:
   a. introducing into non-selective host cells for recombination:
      (i) a pox virus which lacks the function of the 29K gene or portion thereof which is required for replication of the pox virus in selective host cells; and
      (ii) a donor vector for recombination with the pox virus, the donor plasmid comprising:
         A. pox virus DNA sequences containing the 29K gene or a portion thereof sufficient to restore 29K function in the pox virus;
         B. a foreign gene to be inserted into the virus, wherein both the foreign gene and the pox virus DNA are under the direction of a pox viral promoter; and
         C. flanking pox viral sequences substantially homologous to a region of a pox virus genome;
   b. maintaining the cells obtained in step (a) under conditions which permit recombination between the pox virus and the donor vector to provide recombinant virus capable of replicating in the selective host cell; and
   c. selecting for the recombinant virus by its ability to form plaques on the selective host cell.

3. A method of Claim 1 or claim 2, wherein the selection step further comprises plating the viruses resulting from the recombination step onto a lawn of the selective host cells and identifying viral plaques formed on the lawn.

4. A method of Claim 1 or claim 2 wherein the pox virus is vaccinia virus.

5. A method of Claim 1 or claim 2 wherein the selective host cells are RK13 cells.

6. A method of Claim 2, wherein the foreign gene encodes a viral, bacterial or mycobacterial antigen.

7. A method of Claim 6 wherein the foreign gene comprises the gene encoding Hepatitus B surface antigen; or the foreign gene comprises the gene encoding pseudorabies glycoprotein 50 antigen.

8. A method for selecting for recombinant vaccinia virus, comprising:
   a. infecting a non-selective cell with a mutant vaccinia virus which lacks 29K gene function and consequently is unable to replicate on RK13 cells;
   b. transfecting the host cell with a donor plasmid for recombination with the virus, the donor plasmid comprising:
      (i) vaccinia viral DNA sequences containing the 29K gene or a portion thereof sufficient to restore 29K function to the vaccinia virus, and a foreign gene to be inserted into the vaccinia virus, wherein the viral DNA sequences and the foreign gene are under the control of a vaccinia viral promoter; and
      (ii) vaccinia viral flanking sequences homologous to the region of the vaccinia viral genome;
   c. maintaining the host cell under conditions which permit recombination between the vaccinia virus and donor plasmid; and
   d. selecting for recombinant vaccinia virus based upon the ability of the recombinant vaccinia virus to form plaques on RK13 cells.

9. A DNA vector for in vivo recombination with a pox virus to produce a recombinant pox virus capable of expressing a foreign gene, which comprises:
   a. a foreign gene or portion thereof;
   b. pox virus DNA sequences containing the 29K gene, or a portion thereof which is required for replication of the pox virus on a host cell;
   c. one or more pox viral promoters; and
   d. DNA sequences derived from the pox virus, which sequence flank the combined elements a-c at both 5' and 3' ends, wherein the flanking sequences allow recombination into a region of the pox virus which does not inhibit the ability of the virus to replicate.

10. A DNA vector of Claim 9 which is a plasmid.

11. A DNA vector of Claim 9, wherein the foreign gene encodes a viral, bacterial or mycobacterial antigen.

**Patentansprüche**

1. Ein Verfahren zur Selektion von rekombinanten Pockenviren, das die Verfahrensschritte umfaßt:
   a) Rekombinieren eines Pockenvirus, dem die Funktion des 29K-Gens fehlt, oder eines Abschnitts desselben, der zur Replikation des Pockenvirus in selektiven Wirtszellen erforderlich ist, für die das 29K-Gen zur Replikation erforderlich ist, und eines Donor-DNA-Vektors, wobei der Donor-DNA-Vektor (i) eine DNA-Sequenz, die ein fremdes Gen codiert (ii) Pockenvirus-DNA-Sequenzen, die das 29K-Gen oder einen Abschnitt desselben, der zur Rekonstruktion der 29K-Funktion in dem Pockenvirus ausreichend ist, enthalten, und (iii) flankierende Pockenvirus-DNA-Sequenzen, die zu einem Bereich des Pockenvirus-Genoms homolog sind, umfaßt; und
   b) Selektieren von rekombinanten Pockenviren auf der Basis der Fähigkeit des Virus, sich auf den Wirtszellen zu replizieren.

2. Ein Verfahren zur Selektion von rekombinanten Pockenviren, das folgendes umfaßt:
   a) Einführen in nicht-selektive Wirtszellen zur Rekombination:
      (i) eines Pockenvirus, dem die Funktion des 29K-Gens fehlt oder eines Abschnitts desselben, der zur Replikation des Pockenvirus in selektiven Wirtszellen erforderlich ist; und
      (ii) eines Donor-Vektors zur Rekombination mit dem Pockenvirus, wobei das Donor-Plasmid folgendes umfaßt:
         (A) Pockenvirus-DNA-Sequenzen, die das 29K-Gen enthalten oder eines Abschnitts derselben, der ausreichend ist, um die 29K-Funktion in dem Pockenvirus zu rekonstruieren;
         (B) ein Fremdgen, das in das Virus eingebaut werden soll, wobei sowohl das Fremdgen als auch die Pockenvirus-DNA unter der Leitung eines Pockenvirus-Promotors stehen; und
         (C) flankierende Pockenvirus-Sequenzen, die im wesentlichen homolog zu einem Bereich eines Pockenvirus-Genoms sind;
   b) Halten der in Schritt (a) erhaltenen Zellen unter Bedingungen, die Rekombination zwischen dem Pockenvirus und dem Donor-Vektor gestatten, um rekombinante Viren zu liefern, die zur Replikation in der selektiven Wirtszelle fähig sind; und
   c) Selektieren des rekombinanten Virus durch seine Fähigkeit, Plaques auf der selektiven Wirtszelle zu bilden.

3. Ein Verfahren nach Anspruch 1 oder Anspruch 2, bei dem der Selektionsschritt weiterhin das Plattieren der von dem Rekombinationsschritt herrührenden Viren auf einem Rasen von den selektiven Wirtszellen und das Identifizieren von auf dem Rasen gebildeten Virus-Plaques umfaßt.

4. Ein Verfahren nach Anspruch 1 oder Anspruch 2, bei dem das Pockenvirus Vaccinia-Virus ist.

5. Ein Verfahren nach Anspruch 1 oder Anspruch 2, bei dem die selektiven Wirtszellen RK13-Zellen sind.

6. Ein Verfahren nach Anspruch 2, bei dem das Fremdgen ein Virus-, bakterielles oder mycobakterielles Antigen codiert.

EP 0 422 072 B1

7. Ein Verfahren nach Anspruch 6, bei dem das Fremdgen das Gen umfaßt, das Hepatit s-B-Oberflächenantigen codiert, oder das Fremdgen das Gen umfaßt, das Pseudorabies-Glycoprotein-50-Antigen codiert.

8. Ein Verfahren zur Selektion von rekombinantem Vaccinia-Virus, das folgendes umfaßt:

a) Infizieren einer nicht-selektiven Zelle mit einem mutanten Vaccinia-Virus, dem 29K-Gen-Funktion fehlt und das sich demzufolge nicht auf RK13-Zellen replizieren kann;

b) Transfektion eines Donor-Plasmids an die Wirtszelle zur Rekombination mit dem Virus, wobei das Donor-Plasmid folgendes umfaßt:

(i) Vaccinia-Virus-DNA-Sequenzen, die das 29K-Gen oder einen Abschnitt desselben, der ausreichend ist, um die 29K-Funktion an dem Vaccinia-Virus zu rekonstruieren, enthalten, und ein Fremdgen, das in das Vaccinia-Virus eingebaut werden soll, wobei die Virus-DNA-Sequenzen und das Fremdgen unter der Steuerung eines Vaccinia-Virus-Promotors stehen; und

(ii) Vaccinia-Virus-Seitensequenzen, die homolog zu dem Bereich des Vaccinia-Virus-Genoms sind;

c) Halten der Wirtszelle unter Bedingungen, die Rekombination zwischen dem Vaccinia-Virus und dem Donor-Plasmid gestatten; und

d) Selektieren des rekombinanten Vaccinia-Virus auf der Basis der Fähigkeit des rekombinanten Vaccinia-Virus, Plaques auf RK13-Zellen zu bilden.

9. Ein DNA-Vektor für in-vivo-Rekombination mit einem Pockenvirus, um einen rekombinanten Pockenvirus zu erzeugen, der in der Lage ist, ein Fremdgen auszudrücken, der folgendes umfaßt:

a) ein Fremdgen oder einen Abschnitt desselben;

b) Pockenvirus-DNA-Sequenzen, die das 29K-Gen enthalten, oder einen beschnitt desselben, der zur Replikation des Pockenvirus auf einer Wirtszelle erforderlich ist;

c) einen oder mehrere Pockenvirus-Promotoren und

d) DNA-Sequenzen, die von dem Pockenvirus abgeleitet sind, wobei die Sequenz die kombinierten Elemente a-c an beiden 5' und 3'-Enden flankiert und wobei die flankierenden Sequenzen Rekombination in einen Bereich des Pockenvirus gestatten, der nicht die Fähigkeit des Virus zur Replikation behindert.

10. Ein DNA-Vektor nach Anspruch 9, der ein Plasmid ist.

11. Ein DNA-Vektor nach Anspruch 9, bei dem das Fremdgen ein Virus-, bakterielles oder mycobakterielles Antigen codiert.

## Revendications

1. Procédé de sélection de virus vaccins recombinés, comprenant les étapes consistant

a. à recombiner un virus vaccin qui est dénué de la fonction du gène 29K ou d'une partie de celui-ci nécessaire pour la réplication du virus vaccin dans des cellules hôtes sélectives pour lesquelles le gène 29K est nécessaire pour une réplication, et un vecteur ADN donneur, le vecteur ADN donneur comprenant (i) une séquence d'ADN codant pour un gène étranger, (ii) des séquences d'ADN de virus vaccin contenant le gène 29K ou une partie de celui-ci suffisante pour rétablir la fonction du gène 29K dans le virus vaccin, et (iii) des séquences adjacentes d'ADN de virus vaccin homologues d'une région du génome du virus vaccin; et

b. à sélectionner des virus vaccins recombinés sur la base de l'aptitude du virus à se répliquer sur les cellules hôtes.

2. Procédé de sélection de virus vaccins recombinés, comprenant les étapes consistant

a. à introduire dans des cellules hôtes non sélectives, à des fins de recombinaison,

(i) un virus vaccin qui est dénué de la fonction du gène 29K ou d'une partie de celui-ci, nécessaire pour la réplication du virus vaccin dans des cellules hôtes sélectives; et

(ii) un vecteur donneur propre à la recombinaison avec le virus vaccin, le plasmide donneur comprenant:

A. des séquences d'ADN de virus vaccin contenant le gène 29K ou une partie de celui-ci suffisante pour rétablir la fonction du gène 29K dans le virus vaccin;

B. un gène étranger à insérer dans le virus, le gène étranger et l'ADN du virus vaccin étant l'un et l'autre sous la direction d'un promoteur de virus vaccin; et

17

C. des séquences de virus vaccin adjacentes, pratiquement homologues d'une région d'un génome de virus vaccin:

b. à maintenir les cellules obtenues dans l'étape (a) dans des conditions qui permettent une recombinaison entre le virus vaccin et le vecteur donneur, pour obtenir un 'virus recombiné capable de se répliquer dans la cellule hôte sélective; et

c. à sélectionner le virus recombiné sur la base de son aptitude à former des plaques sur la cellule hôte sélective.

3. Procédé selon la revendication 1 ou 2, dans lequel l'étape de sélection comprend en outre l'opération consistant à ensemencer les virus résultant de l'étape de recombinaison sur un parterre des cellules hôtes sélectives et à identifier les plaques virales formées sur le parterre.

4. Procédé selon la revendication 1 ou 2, dans lequel le virus vaccin est le virus de la vaccine.

5. Procédé selon la revendication 1 ou 2, dans lequel les cellules hôtes sélectives sont des cellules RK13.

6. Procédé selon la revendication 2, dans lequel le gène étranger code pour un antigène viral, bactérien ou mycobactérien.

7. Procédé selon la revendication 6, dans lequel le gène étranger comprend le gène codant pour l'antigène de surface de l'hépatite B ou le gène étranger comprend le gène codant pour l'antigène de glycoprotéine 50 de la maladie d'Aujesky.

8. Procédé de sélection de virus recombiné de la vaccine, comprenant les étapes consistant

a. à infecter une cellule non sélective avec un virus mutant de la vaccine qui est dénué de la fonction du gène 29K et, en conséquence, est incapable de se répliquer sur des cellules RK13;

b. à transfecter la cellule hôte avec un plasmide donneur en vue de la recombinaison de celui-ci avec le virus, le plasmide donneur comprenant:

(i) des séquences d'ADN du virus de la vaccine contenant le gène 29K ou une partie de celui-ci suffisante pour rétablir la fonction du gène 29K du virus de la vaccine, et un gène étranger à insérer dans le virus de la vaccine, les séquences d'ADN viral et le gène étranger étant sous la direction d'un promoteur de virus de la vaccine; et

(ii) des séquences adjacentes du virus de la vaccine, homologues de la région du génome du virus de la vaccine;

c. à maintenir la cellule hôte dans des conditions qui permettent la recombinaison entre le virus de la vaccine et le plasmide donneur; et

d. à sélectionner le virus recombiné de la vaccine sur la base de la capacité du virus recombiné de la vaccine à former des plaques sur les cellules RK13.

9. Vecteur ADN propre à la recombinaison in vivo avec un virus vaccin pour produire un virus vaccin recombiné capable d'exprimer un gène étranger, comprenant:

a. un gène étranger ou une partie de celui-ci;

b. des séquences d'ADN de virus vaccin, contenant le gène 29K ou une partie de celui-ci qui est nécessaire pour la réplication du virus vaccin sur une cellule hôte;

c. un ou plusieurs promoteurs de virus vaccin; et

d. des séquences d'ADN dérivées du virus vaccin qui sont adjacentes aux éléments a-c combinés aux deux extrémités 5' et 3', ces séquences adjacentes permettent la recombinaison dans une région du virus vaccin qui n'inhibe pas l'aptitude du virus à se répliquer.

10. Vecteur ADN selon la revendication 9, qui est un plasmide.

11. Vecteur ADN selon la revendication 9, dans lequel le gène étranger code pour un antigène viral, bactérien ou mycobactérien.

A. NYCBH

Hindlll Map

M (2.2 KB)    K (4.5 KB)

55K    30K    29K    10K

B. vABT33

55K    29K    10K

LacZ    BamF

C. vABT71

55K    (deleted)

LacZ    BamF

FIGURE 1

Plasmid & Virus Used for Recombination:

Virus VAbT 33:

Plasmid PAb1005SAG

Phenotype:

$29K^-, RK13^-, LacZ^+, SAG^-$

Phenotype of Recombinant

$29K^+, PK13^+, LacZ^-, SAG^+$

FIGURE 2

EP 0 422 072 B1

PLASMID MAP

AbT Name: pAbT 4081

Plasmid Name: ptk 7.5K gp50 29K

Size: 7335 bp

Vector Fragment: pAbT 4018 SacI, T4 polymerase, partial EcoRI 59s

Insert Fragment: pAbT 4078 PstI, T4 polymerase, EcoRI ~1400 b

Comments: test IVR vector for 29K/RK13 selection at TK

FIGURE 3

21

FIGURE 4

FIGURE 5

SacI   SacI

env

Lambda SIV₂₅₁

1. SacI

pEMBL18

amp

SacI

1. SacI

Ligate

Figure 6A

DT210

XbaI          env →
TCTAGACCATGGGATGTCTTGGGATCAG
AGATCTGGTACCCTACAGAACCCTTAGTC
      NcoI

DT211

PvuII      PvuII  PvuII

env

pAbT
4566

SacI      SacI

PvuII

PvuII

amp

1. partial PvuII

Figure 6B

Ligate

PvuII   PvuII

XbaI,NcoI          PvuII

env

pAbT
4096

SacI

PvuII

amp

1.XbaI + SacI

BamHI

XbaI,BamHI,SmaI  BamF      lacZ
KpnI,SacI

40K  pAbT
     4537

SacI

tk           tk

amp

1. partial SacI
2. XbaI

Ligate

Figure 6C

SacI

BamF    BamHI

env

XbaI,NcoI    pAbT    lacZ
40K   4572

SacI

tk           tk

amp

FIGURE 6

24

FIGURE 7

Figure 8.A

Figure 8.B

Figure 8.C

FIGURE 8